# EUROPEAN PATENT APPLICATION

(11) **EP 4 699 615 A1**
(43) Date of publication of application: **25.02.2026**
(21) Application number: 24195766.1
(22) Date of filing: 22.08.2024
(51) Int. Cl.: A61K 38/19, A61K 38/20, A61K 39/395, A61P 25/00

(54) **FCRN INHIBITOR AND THERAPEUTIC POLYPEPTIDE**

(71) Applicant: Universität Zürich, 8006 Zürich (CH)
(72) Inventor: vom Berg, Johannes, 8051 Zürich (CH); Beffinger, Michal, 8952 Schlieren (CH); Taskoparan, Betül, 8050 Zürich (CH)
(74) Representative: Hepp Wenger Ryffel AG

(57) **Abstract**

An FcRn inhibitor, wherein FcRn is a neonatal fragment crystallisable receptor, for use in reducing, preferably preventing, the transfer, in particular the FeRn-mediated transcytosis, of a therapeutic polypeptide from the central nervous system into the systemic circulation, □wherein the therapeutic polypeptide comprises at least one region selected from the group consisting of: an Fc-region of IgG, an FcRn-binding region of albumin, and an albumin-binding region, wherein the Fc-region is a crystallisable fragment region; and wherein the FcRn inhibitor is delivered by systemic administration and the therapeutic polypeptide is delivered by localised administration, preferably by localised central nervous system administration.

## Description

The present invention relates to an FcRn inhibitor for use in reducing the transfer of a therapeutic polypeptide from the central nervous system into the systemic circulation. It further relates to a therapeutic polypeptide for use in a combination therapy in the prevention or treatment of a disease affecting the central nervous system. It also relates to a combination of an FcRn inhibitor and a therapeutic polypeptide.

Cytokine therapies (e.g. Interleukin-12 (IL-12) for brain tumours, IL-10 for multiple sclerosis (MS)), as well as neutralising antibodies (e.g. against IL-12/23p40 in MS, or against tumour necrosis factor alpha (TNFα) in Parkinson's Disease and Alzheimer's Disease) or immune checkpoint blocking molecules (e.g. blocking PD-1/PD-L1 axis in brain tumours) are used to treat diseases that affect the central nervous system.

Therapeutic agents for the treatment of diseases affecting the central nervous system, such as brain tumours, are often required in high concentrations in the diseased tissue. However, certain therapeutic agents tend to be transported either passively or actively across the blood-brain barrier (or other barrier) from the central nervous system into the systemic circulation. One disadvantage of this is that the concentration of the therapeutic agent in the diseased tissue decreases. The administered dose must therefore be increased in order to maintain the target concentration in the diseased tissue. On the other hand, this has the disadvantage that the administered therapeutic agent can cause undesirable side effects outside the central nervous system.

To give an example, IL-12 is used as a pro-inflammatory cytokine in the treatment of brain tumours. However, systemic, intravenous administration of IL-12 has proven to be particularly detrimental, as high systemic levels of IL-12 cause severe side effects (e.g. high systemic levels of interferon gamma (IFNγ)). Local administration (e.g. by convection-enhanced delivery (CED)) can reduce the extent of this problem. However, IL-12 is still transported from the central nervous system by passive leakage. To further minimise the problem, fusion proteins are also produced, such as IL-12Fc. IL-12Fc is produced from IL-12 and the crystallisable fragment (Fc) of immunoglobulin G (IgG). IL-12Fc, for example, shows increased pharmacostability, bioavailability and a reduced passive leakage from the central nervous system compared to IL-12. However, the reduced passive leakage into the systemic circulation is negatively compensated by the active transport of IL-12Fc through the blood-brain barrier mediated by the neonatal Fc receptor (FcRn). Due to the active transport of IL-12Fc through the endothelial cells of the blood-brain barrier by FcRn, the systemic accumulation of IL-12Fc is enhanced when administered locally to the central nervous system. As a result, the therapeutic window in which such therapeutic agents can be used is very small.

The problem is of course not limited to IL-12Fc, but also to other therapeutic agents whose systemic presence is undesirable and which pass through the blood-brain barrier from the central nervous system, for example through FcRn-mediated transcytosis. In addition, the problem does not only occur with therapeutics that comprise an Fc region of IgG, but also with other therapeutics that are affected by FcRn-mediated transport. These include therapeutics that comprise an FcRn-binding part of albumin or may have an albumin-binding moiety.

In the prior art, the problem is solved, for instance, by modifying the FcRn-binding component(s) so that the affinity to FcRn is reduced and thus the active transport by FcRn through the barriers of the central nervous system is limited. This is described in COOPER, P. R. et al. (Efflux of monoclonal antibodies from rat brain by neonatal Fc receptor, FcRn. Brain. Res. 2013, Vol. 1534, pages 13 to 21), for example. There it is shown that a change in the affinity to FcRn of IgG1 caused by mutations has a strong influence on the efflux rate from the central nervous system.

The disadvantage of this solution is that mutations have to be inserted into the therapeutic agent. Firstly, this can be very resource-intensive and care must be taken to ensure that the therapeutic effect is not negatively influenced. Furthermore, the choice of suitable mutations for that purpose can be challenging.

It is therefore an object of the present invention to overcome at least some, if not all shortcomings of the prior art methods. In particular, it is an object of the invention to provide means to reduce, preferably prevent, the transfer, in particular the FcRn-mediated transcytosis, of a therapeutic agent from the central nervous system into the systemic circulation.

The problem is solved by an FcRn inhibitor for use in reducing the transfer of a therapeutic polypeptide from the central nervous system into systemic circulation; by a therapeutic polypeptide for use in a combination therapy in the prevention or treatment of a disease affecting the central nervous system; and by a combination therapy for use in a combination therapy in the prevention or treatment of a disease affecting the central nervous system according to the independent claims. Advantageous embodiments and further developments are the subject of the dependent claims.

A first aspect of the invention relates to an FcRn inhibitor, wherein FcRn is a neonatal fragment crystallisable receptor, for use in reducing, preferably preventing, the transfer, in particular the FcRn-mediated transcytosis, of a therapeutic polypeptide from the central nervous system into the systemic circulation. The therapeutic polypeptide comprises at least one region selected from the group consisting of: an Fc-region of IgG, an FcRn-binding region of albumin, and an albumin-binding region. The Fc-region is a crystallisable fragment region. The FcRn inhibitor is delivered by systemic administration. The therapeutic polypeptide is delivered by localised administration, preferably by localised central nervous system administration.

The neonatal Fc receptor (FcRn) is a specialised receptor that is involved in the regulation of the levels of immunoglobulin G (IgG) antibodies and serum albumin in the bloodstream. It is structurally similar to major histocompatibility complex (MHC) class I molecules and is composed of an alpha chain and beta-2 microglobulin. FcRn is expressed in various tissues, including endothelial and epithelial cells. Its primary function is to bind IgG at acidic pH levels within early endosomes, protecting these antibodies from lysosomal degradation, and then release them at neutral pH back into the circulation. Similarly, the lysosomal degradation of serum albumin is reduced by FcRn. For detailed descriptions of FcRn, its structure and functions, it is referred to PYZIK, M. et al. (The Neonatal Fc Receptor (FcRn): A Misnomer? Front. Immunol. 2019, Vol. 10, Article 1540).

In the context of the present invention, the term "or" is to be understood as an exclusionary disjunction. "A or B" states that exactly one of the two statements is true (if the disjunction is true).

A therapeutic polypeptide is an engineered or natural polypeptide for the treatment of a disease by modulating biological processes. Such a polypeptide can for example mimic, enhance, or inhibit natural proteins' functions in the body. It comprises at least one target-binding region. The therapeutic antibody is capable of specific binding to a target (wherein a target can for example be a protein or another molecule) with a high affinity, preferably with an equilibrium dissociation constant K_{D} for the binding affinity for the target of at most 100 nM, preferably at most 50 nM, more preferably at most 10 nM, even more preferably at most 1 nM, most preferably at most 0.1 nM, as measured by surface plasmon resonance (SPR, see below). The desired therapeutic function is performed by binding to the target. Essentially, any therapeutic polypeptide according to the invention that is susceptible to undesirable transfer from the central nervous system into the systemic circulation, in particular by FcRn-mediated transcytosis, can be considered.

The dissociation constant K_{D} refers to an equilibrium constant that is a measure for the propensity of a complex composed of (mostly two) different components to dissociate reversibly into its constituent components, and is expressed in molar concentration (mol/l, M). The dissociation constant can be calculated according to the formula K_{D} = ([A]×[B])/[AB], wherein [AB] is the concentration of the complex AB, [A] is the concentration of component A and [B] is the concentration of component B. For example, component A can be a therapeutic polypeptide and component B can be the pharmaceutical target of component A, wherein the dissociation constant would be a measure for the binding affinity of the therapeutic polypeptide for the pharmaceutical target. In another example, component A can be the therapeutic polypeptide and component B can be FcRn, wherein the dissociation constant would denote the binding affinity for the therapeutic polypeptide towards FcRn. The lower the binding affinity, the higher the K_{D} value, and vice versa. K_{D} is typically determined using methods well established in the art (e.g. SPR, see below).

In the context of the present invention, the term `crystallisable fragment (Fc) region' refers to a fraction of an IgG antibody comprising two identical heavy chain fragments covalently linked by disulfide bonds or to a single heavy chain fragment. The heavy chain fragments are comprised of constant domains (a constant heavy chain 2 (CH2) and a constant heavy chain 3 (CH3) domain in IgG antibody isotypes). IgG is a major effector of the humoral immune response. There are four distinct subgroups of human IgG designated IgG1, IgG2, IgG3 and IgG4. The four subclasses show more than 95 % homology in the amino acid sequences of the constant domains of the heavy chains, but differ with respect to structure and flexibility of the hinge region, especially in the number of inter-heavy chain disulfide bonds in this domain. The structural differences between the IgG subclasses are also reflected in their susceptibility to proteolytic enzymes, particularly papain, plasmin, trypsin and pepsin.

Preferably, the Fc-region of IgG is an Fc-region of human IgG1, human IgG2, human IgG3 or human IgG4, more preferably an Fc-region of human IgG1, human IgG2 or human IgG4, most preferably an Fc-region of human IgG4. Preferably, the form of IgG is recombinant IgG.

In the context of the present invention, a therapeutic polypeptide comprising an Fc region of IgG (immunoglobulin G) is a polypeptide, preferably a fusion polypeptide, wherein the target-binding region is fused (directly or indirectly) to the Fc region and/or comprises the Fc region. The Fc region contains at least a part of an Fc fragment of an IgG antibody. The IgG residues known to be involved in FcRn binding of IgG1, IgG2 and IgG4 are isoleucine 253 (I253), histidine 310 (H310), and histidine 435 (H435) (located at the CH2-CH3 domain interface. Human IgG3 bears an arginine (R) at position 435. These residues involved in FcRn binding as well as the pH dependence of the interaction between these residues and FcRn are known from the prior art (e.g. in PYZIK, M. et al. The Neonatal Fc Receptor (FcRn): A Misnomer? Front. Immunol. 2019, Vol. 10, Article 1540; SHIELDS, R. L. et al. High Resolution Mapping of the Binding Site on Human IgG1 for FcgRI, FcgRII, FcgRIII, and FcRn. JBC 2001, Vol. 276, No. 9, pages 6591 to 6604).

In the context of the present invention, the EU numbering system (EDELMAN, G. M. et al. The covalent structure of an entire gamma-Immunoglobulin molecule. Proc. Natl. Acad. Sci. USA 1969, Vol. 63, No. 1, pages 78 to 85) is used for the numbering of amino acid residues in the Fc region. The EU numbering scheme is a widely adopted standard for numbering the residues in an antibody in a consistent manner.

In the context of the present invention, a therapeutic polypeptide comprising an FcRn-binding region of albumin is a polypeptide, preferably a fusion polypeptide, wherein the target-binding region is fused (directly or indirectly) to the FcRn-binding region of albumin and/or comprises the FcRn-binding region of albumin. The principal binding site for FcRn on albumin is known to be located within the C-terminal DIII. Within DIII of human albumin, the histidine residues H464, H510 and H535, the lysine residue K500 are highly relevant for binding, among other residues. The FcRn-binding region(s) of albumin is known from the prior art (e.g. in KNUDSEN SAND, K. M. et al. Unraveling the interaction between FcRn and albumin: opportunities for design of albumin-based therapeutics. Front. Immunol. 2015, Vol. 5, article 682). Residue H464 seems to be particularly relevant for FcRn binding (ANDERSEN, J. T. et al. Structure-based mutagenesis reveals the albumin-binding site of the neonatal Fc receptor. Nat. Comm. 2012, Vol. 3, article 610; MOMIN, N. et al. Maximizing response to intratumoral immunotherapy in mice by tuning local retention. Nat. Commun. 2022, Vol. 13, article 109). Preferably, the albumin is human serum albumin and/or recombinant albumin. The therapeutic polypeptide comprising an FcRn-binding region of albumin can also include an entire albumin molecule.

In the context of the present invention, a therapeutic polypeptide comprising an albumin-binding region is a polypeptide, preferably a fusion polypeptide, wherein the target-binding region is fused (directly or indirectly) to a region that binds to albumin and/or comprises a region that binds to albumin.

The Fc region of IgG and/or the FcRn-binding region of albumin of the present invention may also be modified such that their protein sequence deviates from the naturally occurring Fc region of IgG or the naturally occurring FcRn-binding region of albumin, but preferably only such that the binding affinity of the therapeutic polypeptide to FcRn does not change significantly. Preferably, the equilibrium dissociation constant K_{D} for FcRn of the therapeutic polypeptide changes by at most 30 %, preferably by at most 20 %, most preferably by at most 10 %as determined by surface plasmon resonance (SPR, see below) at pH 6.0.

The differences and similarities between different FcRn types (e.g. human, rat or mouse) are known in the art, as are the differences and similarities between distinct types of Fc regions of IgG, and distinct types of albumin. Furthermore, it is known in the art that the binding affinity of the therapeutic polypeptide to FcRn is an intrinsic property of the Fc region of IgG (and/or the FcRn-binding region of albumin), which is defined by the corresponding amino acid sequence, independent of a polypeptide linked to it. Similarly, it is known that the part of the therapeutic polypeptide exhibiting a therapeutic effect can be attached to the Fc region of IgG, the FcRn-binding region of albumin and/or the albumin-binding region without adversely affecting the therapeutic properties. The functional domains responsible for the therapeutic effects of the therapeutic polypeptide are to be designed to be distinct from the attachment site of the Fc region of IgG, the FcRn-binding region of albumin and/or the albumin-binding region. This ensures that the therapeutically active site and the FcRn-binding site are not (adversely) affected by each other, unless such an influence is desired. Fc-fusions and albumin-fusions are a well-established field (CZAJKOWSKY, D. M. et al. Fc-fusion proteins, new developments and future perspectives. EMBO MM 2012, Vol. 4, pages 1015 to 1028; SLEEP, D. Albumin and its application in drug delivery, Expert. Opin. Drug Deliv. 2014, Vol. 12, No. 5, pages 793 to 812; TAO, H. et al. The development of human serum albumin-based drugs and relevant fusion proteins for cancer therapy. Int. J. Biol. Macromol. 2021, Vol 187, pages 24 to 34). Specific examples of therapeutic polypeptides are given below.

In the context of the present invention, localised administration refers to the targeted delivery of a therapeutic agent directly to a specific area of the body, such as a particular tissue, organ, or site of disease, with the goal of maximising the therapeutic effect at that location while minimising systemic exposure and potential side effects.

In the context of the present invention, localised central nervous system administration refers to the direct delivery of therapeutic agents into the central nervous system, bypassing the barriers of the central nervous system, such as the blood-brain barrier. The administration may be continuous, intermittent, or nonrecurring. The expression "localised central nervous system administration" is also meant to include rinsing of a resection cavity following an operation.

With regard to the present invention, in absence of an FcRn inhibitor, a therapeutic polypeptide comprising an Fc region of IgG and/or an FcRn-binding region of albumin is negatively affected by FcRn (in particular, when the therapeutic polypeptide is delivered locally to the central nervous system). Endothelial FcRn can bind to the Fc region of IgG or the FcRn-binding region of albumin in these polypeptides at the acidic pH within endosomes. This leads to the transcytosis of the therapeutic polypeptide across the barriers of the central nervous system (e.g. the blood-brain barrier, BBB), subsequently leading to their unintended transport into the systemic circulation. The same applies to a therapeutic polypeptide with an albumin-binding region. As soon as this polypeptide binds to albumin, the resulting complex has the potential to be transferred into the systemic circulation due to the albumin.

In the context of the present invention, the transfer from the central nervous system into the systemic circulation is a process in which the therapeutic polypeptide is at least partially transferred from the central nervous system into the systemic circulation, either by active or passive transport mechanisms, preferably active transport mechanisms. In the context of the present invention, FcRn-mediated transcytosis is the transcellular transport process of engulfing a substance in a vesicle on one side of a barrier of the central nervous system (e.g. the blood-brain barrier) and releasing them on the other side.

In the context of the present invention, the systemic circulation refers to all parts of a biological body outside the central nervous system, encompassing organs, tissues and blood vessels that constitute the circulatory system responsible for transporting blood, nutrients, and other substances throughout the body. This includes the heart, arteries, veins and capillaries, as well as the peripheral organs such as the liver, kidneys, lungs, and muscles.

To prevent this undesired transfer, an FcRn inhibitor is administered systemically. In the context of the present invention, an FcRn inhibitor refers to a compound (or a group of compounds) that binds to FcRn and prevents or reduces interaction of FcRn with other binding partners of FcRn (e.g. IgG antibodies, albumin, or a therapeutic polypeptide according to the invention). This inhibition particularly disrupts the undesired transfer of the therapeutic polypeptide (especially when administered locally) from the central nervous system into the systemic administration. Essentially any FcRn inhibitor according to the invention that is capable of blocking FcRn, in particular endothelial FcRn at a barrier of the central nervous system, is suitable. The FcRn inhibitor can also be referred to as FcRn blocker or FcRn receptor antagonist. Preferably, the inhibition activity is increased or is only substantially present at a pH below 7.0, preferably at a pH of at most 6.0. The FcRn inhibitor is preferably administered before the therapeutic polypeptide, but can also be administered at the same time or at a later time. The FcRn inhibitor can be administered in one dose at a time or continuously or in portions. Specific examples of possible FcRn inhibitors are given below.

The reduction or prevention of transfer of the therapeutic polypeptide from the central nervous system to the systemic circulation, particularly by mechanisms such as FcRn-mediated transcytosis, can be determined by multiple methods known in the art. For example, the concentration of the therapeutic polypeptide in the systemic circulation (i.e. in the blood serum and/or blood plasma) and in the brain can be determined by Enzyme-Linked Immunosorbent Assay (ELISA), as described in more detail below. Comparing the ratio between systemic levels and levels in the central nervous system with and without administering an FcRn inhibitor indicates then a degree of reduction. Preferably, the serum to brain concentration ratio or the plasma to brain concentration ratio of the therapeutic polypeptide, after administration of an FcRn inhibitor, is smaller by a factor of at most 0.8, preferably at most 0.6, more preferably at most 0.4, even more preferably at most 0.3, most preferably at most 0.2 compared to the serum to brain concentration ratio or the plasma to brain concentration ratio of the therapeutic polypeptide without the administration of an FcRn inhibitor, measurable after a predetermined period of time, preferably 24 h, after convection-enhanced delivery (CED) of the therapeutic polypeptide into the right striatum of hFcRn Tg32 mice, wherein an FcRn inhibitor has been administered intravenously 24 h before the therapeutic polypeptide. Reference is made to ROOPENIAN, D. C. et al. (Human FcRn Transgenic Mice for Pharmacokinetic Evaluation of Therapeutic Antibodies. In: Proetzel, G., Wiles, M. (eds) Mouse Models for Drug Discovery. Methods in Molecular Biology, vol 602. Humana Press, 2010) and PETKOVA, S. B. et al. (Enhanced half-life of genetically engineered human IgG1 antibodies in a humanized FcRn mouse model, potential application in humorally mediated autoimmune disease. Int. Immunol. 2006, Vol. 18, No. 12, pages 1759 to 1769) for protocols on hFcRn Tg32 mice.

Administration of the FcRn inhibitor increases the therapeutic efficacy of the therapeutic polypeptide that has its target(s) within the central nervous system. This is because the concentration of the therapeutic polypeptide is maintained at a higher level in the central nervous system. Conversely, lower administered doses are necessary to reach and maintain a certain level in the central nervous system. In addition, the side effects triggered by the therapeutic polypeptide are reduced, as less of it is retained in the systemic circulation.

Surprisingly, it was found that systemic administration of the FcRn inhibitor leads to a far stronger reduction of transfer of the therapeutic polypeptide from the central nervous system into the systemic circulation, compared to local administration into the central nervous system. For instance, it was found that by local co-administration of the FcRn inhibitor together with the therapeutic polypeptide into the central nervous system, no improvement in retention advantage was detected compared to the administration of the therapeutic polypeptide alone. It was expected that local administration into the central nervous system would lead to a better result (or at least to an equal result).

The exact causes for this surprising effect are not clear. Without wishing to be bound by any theory, one possible explanation for this unexpected result is that the endothelial FcRn at the barriers of the central nervous system, such as the blood-brain barrier, plays a more significant role in mediating the export of therapeutic polypeptides from the brain to the blood than the FcRn present within the parenchyma of the central nervous system. Systemic administration of the FcRn inhibitor may more effectively block the FcRn receptors on the endothelial cells of the blood-brain barrier, thereby reducing the transfer, e.g. the transcytosis, and subsequent clearance of the therapeutic polypeptide into the systemic circulation. In contrast, local administration into the central nervous system might not adequately reach and inhibit the FcRn at these critical barrier sites, resulting in a lesser impact on the retention of the therapeutic polypeptide within the central nervous system. Apart from that, systemic administration of the FcRn inhibitor might result in sustained levels of the FcRn inhibitor in the circulation, providing continuous inhibition of FcRn over a longer period. Local administration could result in a more transient presence of the inhibitor in the central nervous system leading to an incomplete or intermittent inhibition of FcRn. Local administration to the central nervous system might also lead to an uneven distribution of the inhibitor, potentially leaving some areas of the barrier inadequately inhibited.

A particular advantage of the solution according to the invention is that the systemic load of a therapeutic polypeptide, which is supposed to act in the central nervous system, remains low, and the level in the central nervous system remains high, without having to modify the therapeutic polypeptide, e.g. by introducing mutations, specifically for this purpose. Thus, the invention can be applied to many different therapeutic polypeptides which are prone to efflux from the central nervous system, in particular by FcRn-mediated transcytosis. This avoids that any necessary modifications would negatively alter the therapeutic effect of the therapeutic polypeptide. Furthermore, without wishing to be bound by any theory, if a therapeutic polypeptide is used that does not bear a modification to alter the affinity to FcRn, the locally administered therapeutic polypeptide might still interact with FcRn in the parenchyma of the central nervous system. This interaction can have favourable effects. For instance, the binding of the therapeutic polypeptide to FcRn in the central nervous system's parenchyma could protect it from lysosomal degradation, thereby prolonging its half-life and enhancing its therapeutic efficacy within the central nervous system. In case the therapeutic polypeptide is used for cancer treatment in the central nervous system, for example, it can engage with the FcRn expressed by tumours and tumour-infiltrating immune cells, which might enhance its therapeutic effects. It is assumed that the tumoral FcRn is less blocked by the FcRn inhibitor delivered by systemic administration. Further, FcRn binds to antigen-antibody immune complexes (e.g. comprising tumour-associated antigens) and can deliver these complexes to dendritic cells. The dendritic cells in turn activate T cells, which attack the tumour from which the antigens originate. If the parenchymal FcRn at a central nervous system tumour is not blocked (which is assumed in the present invention), the described desired immune response is not inhibited (see also PYZIK, M. et al. The Neonatal Fc Receptor (FcRn): A Misnomer? Front. Immunol. 2019, Vol. 10, Article 1540).

Overall, the therapeutic window of the therapeutic polypeptide is greatly enlarged by the invention. Thus, the invention provides means to reduce, preferably prevent, the transfer, in particular the FcRn-mediated transcytosis, of a therapeutic agent from the central nervous system into the systemic circulation.

A second aspect of the present invention relates to a therapeutic polypeptide for use in a combination therapy in the prevention or treatment of a disease affecting the central nervous system, particularly a primary and secondary brain cancer. The therapeutic polypeptide comprises at least one region selected from the group consisting of: an Fc-region of IgG, an FcRn-binding region of albumin, and an albumin-binding region (the Fc-region is a crystallisable fragment region). The combination therapy further comprises an FcRn-blocking inhibitor (FcRn is a neonatal fragment crystallisable receptor). The inhibitor is delivered by systemic administration. The therapeutic polypeptide is delivered by localised administration, preferably by localised central nervous system administration.

The nature of the therapeutic polypeptide according to the invention has been described above; specific examples will be given below.

Diseases affecting the central nervous system for which the therapeutic polypeptide can be used according to the invention include: inflammation, dementia, stroke, cancer, infectious diseases, cystic fibrosis, multiple sclerosis, Parkinson's disease, a hereditary disorder, rheumatoid arthritis, gout, and pseudogout. The list is not exhaustive. Reference is made to WO 2020/201167 A1 (UNIV ZUERICH, 08 October 2020) and WO 2020/201168 A1 (UNIV ZUERICH, 08 October 2020) from the prior art, in which the diseases mentioned are described in more detail. In addition, the diseases described there relating to the central nervous system are also included in the disclosure of the present patent application.

Preferably, the cancer is selected from the group consisting of:
- brain cancer, preferably selected from the group consisting of a glioma (more preferably selected from the group consisting of an astrocytoma, an oligodendroglioma, and a glioblastoma (GBM)), a medulloblastoma, a meningioma, an ependymoma, and primary central nervous system lymphoma;
- a spinal cord tumour, preferably selected from the group consisting of intramedullary tumours and extramedullary tumours;
- a metastatic brain tumour;
- leptomeningeal carcinomatosis; and
- a primary bone cancer affecting the spinal cord.

Preferably, the cancer is brain cancer, more preferably a glioblastoma (GBM).

Essentially, the same effects and advantages over the prior art can be derived for the second aspect of the invention, which have already been explained in detail with regard to the first aspect. In particular, the systemically administered FcRn inhibitor improves the effect of the therapeutic polypeptide. This is because in this combination therapy the systemic accumulation of the therapeutic polypeptide is reduced or prevented. This leads to fewer side effects of the therapeutic polypeptide outside the central nervous system, as well as to an improved and more stable level of the therapeutic polypeptide in the central nervous system, where it is supposed to exert its effect(s).

A third aspect of the invention relates to a combination of pharmaceutical components for use in a combination therapy in the prevention or treatment of a disease affecting the central nervous system, particularly a primary and secondary brain cancer. The combination comprises an FcRn inhibitor (FcRn is a neonatal fragment crystallisable receptor); and a therapeutic polypeptide comprising at least one region selected from the group consisting of: an Fc-region of IgG, an FcRn-binding region of albumin, and an albumin-binding region (the Fc-region is a crystallisable fragment region). The FcRn inhibitor is delivered by systemic administration. The therapeutic polypeptide is delivered by localised administration, preferably by localised central nervous system administration.

This aspect is to be understood as a kit-of-parts, wherein the components of the kit are prepared in such a way that they can be administered for treatment. The components are stored packaged independently of each other before administration. The components of the kit need not be administered simultaneously; and the FcRn inhibitor is administered systemically and the therapeutic polypeptide is preferably administered locally into the central nervous system.

Again, reference is made to the description of the first and second aspects for the effects and advantages of the invention according to the third aspect.

In a preferred embodiment of any aspect of the invention, the FcRn inhibitor is selected from the group consisting of: an FcRn-blocking antibody or antibody fragment; an FcRn-blocking peptide; and an FcRn-degrading agent, preferably an FcRn-degrading peptide, more preferably an FcRn-degrading proteolysis targeting chimera (PROTAC).

In the context of the present invention, an FcRn-blocking antibody or antibody fragment (such as a Fab, scFv, or Fc fragment) is suitable to bind to FcRn and inhibit its interaction with other binding partners. Specific examples of FcRn-blocking antibodies or antibody fragments are given below. Similarly, in the context of the present invention, an FcRn-blocking peptide is a chain of amino acids suitable to bind to FcRn and inhibit its interaction with other binding partners.

In the context of the present invention, an FcRn-degrading agent, in particular an FcRn-degrading peptide, is a compound that induces the degradation of the FcRn protein, thereby reducing its levels in cells. For example, this can be achieved by promoting the ubiquitination and subsequent proteasomal degradation of FcRn. A PROTAC is a bifunctional molecule that simultaneously binds to the target protein (FcRn in this case) and an E3 ubiquitin ligase. This dual binding facilitates the ubiquitination of FcRn, marking it for degradation by the proteasome.

Similar to FcRn blockade, the FcRn degradation leads to a lower efflux of the therapeutic polypeptide from the central nervous system into the systemic circulation.

The inventors have found that several types of FcRn inhibitors, such as the ones mentioned above, are suitable for preventing the transfer of a therapeutic polypeptide from the central nervous system into the systemic circulation.

In another preferred embodiment of any aspect of the invention, the equilibrium dissociation constant K_{D} for the binding affinity for FcRn of the FcRn inhibitor at pH 6.0, is at most 30 nM, preferably is at most 25 nM, more preferably is at most 20 nM, most preferably is at most 15 nM. Additionally, or alternatively, said equilibrium dissociation constant K_{D} at pH 7.4, is at most 360 nM, preferably is at most 350 nM, more preferably is at most 340 nM, most preferably is at most 330 nM. The equilibrium dissociation constant K_{D} for the FcRn inhibitor is measured by surface plasmon resonance (SPR, wherein FcRn is in the immobilised phase, see details below).

The inventors have found that FcRn inhibitors with such a binding affinity or lower, especially at pH 6.0, are particularly well suited to reduce or even prevent the transfer, in particular the FcRn-mediated transcytosis, of a therapeutic polypeptide from the central nervous system into the systemic circulation.

In a further preferred embodiment of any aspect of the invention, the equilibrium dissociation constant K_{D} for the binding affinity for FcRn of the therapeutic polypeptide at pH 6.0, is at most 750 nM, preferably is at most 650 nM, more preferably is at most 550 nM. Additionally, or alternatively, said equilibrium dissociation constant K_{D} at pH 7.4, is at least 1000 nM, preferably is at least 1100 nM, more preferably is at least 1200 nM.

Again, the equilibrium dissociation constant K_{D} for the therapeutic polypeptide is measured by surface plasmon resonance (SPR, see details below). The values are to be understood in the absence of any FcRn inhibitor. In case of a therapeutic polypeptide comprising an albumin-binding region, the values define the equilibrium dissociation constant K_{D} for the complex comprising the therapeutic polypeptide and albumin.

The inventors have found that therapeutic polypeptides with such a binding affinity to FcRn have a particularly strong tendency to be transferred from the central nervous system into the systemic circulation, in particular by FcRn-mediated transcytosis. As an example, the therapeutic polypeptide IL-12Fc, which has corresponding binding affinities, shows a strong tendency for FcRn-mediated transcytosis into the systemic circulation (see above).

In a further preferred embodiment of any aspect of the invention, the equilibrium dissociation constant K_{D} for the binding affinity for FcRn of the therapeutic polypeptide, at pH 6.0, is at least 5 times higher, preferably at least 10 times higher, most preferably at least 15 times higher than the equilibrium dissociation constant K_{D} for the binding affinity for FcRn of the FcRn inhibitor. In case of a therapeutic polypeptide comprising an albumin-binding region, the values define the equilibrium dissociation constant K_{D} for the complex comprising the therapeutic polypeptide and albumin.

The inventors have found that such a difference in binding affinity between FcRn inhibitor and therapeutic polypeptide is particularly well suited to prevent or reduce the transfer of the therapeutic polypeptide from the central nervous system into the systemic circulation.

In another preferred embodiment of any aspect of the invention, the FcRn is endothelial FcRn at a barrier selected from the group consisting of: a blood-brain barrier, a barrier between spinal cord parenchyma and surrounding vasculature, a cerebrospinal fluid-brain barrier, a blood-cerebrospinal fluid barrier, and a blood-tumour barrier. Preferably, the barrier is a blood-brain barrier.

The central nervous system barriers are described further in SCHELLHAMMER, L. et al. (Exit pathways of therapeutic antibodies from the brain and retention strategies. iScience 2023, Vol. 26, No. 11, article 108132) and STEEG, P. S. (The blood-tumour barrier in cancer biology and therapy. Nat. Rev. Clin. Oncol. 2021, Vol. 18, pages 696 to 714).

The inventors have found that essentially any central nervous system barrier that consists of a risk of efflux of a therapeutic polypeptide is amenable to the invention.

In a further preferred embodiment of any aspect of the invention, the FcRn inhibitor reduces, preferably substantially prevents, the binding of the therapeutic polypeptide with FcRn, preferably at a pH of at most 6.0.

The term reduced binding of the therapeutic polypeptide refers to a measurable decrease in the affinity or interaction between the therapeutic polypeptide and FcRn. In particular, the binding of the therapeutic polypeptide with FcRn is reduced when the equilibrium dissociation constant K_{D} for the binding affinity for FcRn of the therapeutic polypeptide at pH 6.0 is larger than 750 nM in presence of an FcRn inhibitor. The term "substantially prevented binding" refers to a near-complete inhibition interaction between the therapeutic polypeptide and FcRn. In particular, the binding of the therapeutic polypeptide with FcRn is substantially prevented when the equilibrium dissociation constant K_{D} for the binding affinity for FcRn of the therapeutic polypeptide at pH 6.0 is at least 1000 nM in presence of an FcRn inhibitor. Again, K_{D} is measured by SPR. In case of a therapeutic polypeptide comprising an albumin-binding region, the values define the equilibrium dissociation constant K_{D} for the complex comprising the therapeutic polypeptide and albumin.

In another preferred embodiment of any aspect of the invention, the FcRn inhibitor is selected from the group consisting of:
- an antibody or antibody fragment comprising an Fc-region of IgG, wherein the Fc-region is a crystallisable fragment region, comprising a modification resulting in enhanced affinity to the FcRn, preferably, the antibody or antibody fragment is a human IgG1 Fc fragment with MST-HN mutations, more preferably the antibody or antibody fragment is efgartigimod alfa (international non-proprietary name; CAS-No.: 1821402-21-4) or a biosimilar thereof;
- rozanolixizumab (international non-proprietary name; CAS-No.: 1584645-37-3);
- batoclimab (international non-proprietary name: IMVT-1401; CAS-No.: 2187430-05-1);
- nipocalimab (CAS-No.: 2211985-36-1);
- SYN1436;
- SYN1327; and
- SYNT001 (generic name: orilanolimab; DrugBank Accession Number: DB15312).

In general, the FcRn inhibitor can be a biosimilar of the above-mentioned substances, in particular of antibodies or antibody fragments. In the context of the present invention, a biosimilar refers to a biologic product that is highly similar to a reference product, with no clinically meaningful differences in terms of safety, purity, and efficacy. A biosimilar FcRn inhibitor has comparable pharmacokinetics, pharmacodynamics, and clinical effectiveness to the original compound.

Preferably, the modification resulting in enhanced affinity to FcRn is preferably a pH-dependent affinity with a lower affinity (i.e. a higher K_{D} value) at pH 7.4 than at pH 6.0.

A human IgG1 Fc fragment with MST-HN mutations has been modified to comprise the mutations M252Y, S254T, T256E, H433K, N434F. Such Fc fragments as well as efgartigimod alfa are further described in ULRICHTS, P. et al. (Neonatal Fc receptor antagonist efgartigimod safely and sustainable reduces IgGs in humans. J. Clin. Invest. 2018, Vol. 128, No. 10, pages 4372 to 4386).

The above-mentioned types of FcRn inhibitors, in particular efgartigimod alfa, rozanolixizumab, batoxlimab, nipocalimab, SYN1436, SYN1327 and SYNT001, are further described and their chemical structure disclosed in ZHU, L. N. et al. (FcRn inhibitors: a novel option for the treatment of myasthenia gravis. Neural. Regen. Res. 2023, Vol. 18, No. 8, pages 1637 to 1644), in MEZO, A. R. et al. (Reduction of IgG in nonhuman primates by a peptide antagonist of the neonatal Fc receptor FcRn. Proc. Natl. Acad. Sci. USA 2008, Vol. 105, pages 2337 to 2342), in MEZO, M. et al. (Structure-activity relationships of a peptide inhibitor of the human FcRn-human IgG interaction. Bioorg. Med. Chem. 2008, Vol. 16, pages 6394 to 6405), and in BLUMBERG, L. J. et al. (Blocking FcRn in humans reduces circulating IgG levels and inhibits IgG immune complex-mediated immune responses. Sci. Adv. 2019, Vol. 5, article eaax9586). These documents are incorporated herein by reference. SYN1436 and SYN1327 are short amino acid chains acting as FcRn-blocking peptides. Reference is made to SYNT001 is a humanized de-immunized IgG4 monoclonal antibody harbouring a S241P mutation.

According to the inventors, the reduction of the efflux from the central nervous system into the systemic circulation of the therapeutic polypeptide according to the invention can be achieved by a broad number of FcRn inhibitors, administered systemically.

In a further preferred embodiment of any aspect of the invention, the FcRn inhibitor is a small compound. In the context of the present invention, a small compound is a compound, preferably an organic molecule or an organometallic compound, with a molecular weight of at most 2000 g/mol, preferably at most 1500 g/mol, more preferably at most 1000 g/mol.

In a further preferred embodiment of any aspect of the invention, the therapeutic polypeptide is selected from the group consisting of or comprises a member of the group consisting of:
- an immunomodulatory agent, preferably a cytokine, more preferably an interleukin or interleukin derivative, preferably selected from the group consisting of IL-12Fc and IL-10FC;
- an interferon;
- an agonist of a tumour necrosis factor receptor superfamily member;
- a transforming growth factor;
- an immune checkpoint and regulatory protein;
- a colony-stimulating factor;
- a costimulatory molecule;
- a chemokine;
- a growth factor; and
- a neurotrophic factor.

In another preferred embodiment of any aspect of the invention, the therapeutic polypeptide is selected from the group consisting of or comprises a member of the group consisting of: IL-1RA, IL-2, IL-4, IL-5, IL-6, IL-7, IL-8, IL-10, IL-12, IL-13, IL-15, IL-18, IL-21, IL-23, IL-35, interferon alpha (IFNα), interferon beta (IFNβ), interferon gamma (IFNγ), tumour necrosis factor alpha (TNFα), transforming growth factor alpha (TGFα), transforming growth factor beta (TGFβ), transforming growth factor beta receptor II (TGFβRII), signal regulatory protein alpha (SIRPα), granulocyte-macrophage colony-stimulating factor (GM-CSF), granulocyte-colony stimulating factor (G-CSF), CD80, CD86, CD95, GITRL, 4-1BBL, OX40L, EphrinA1, EphrinB2, EphrinB5, C-X-C motif chemokine 10 (CXCL10), CX3CL1, CXCL16, BDNF, GDNF, NRTN, ARTN, PSPN, CNTF, TRAIL, CCL5, CCL10, CCL16, CCL21, IgG1, IgG2, IgG3, IgG4, and albumin, preferably IL-12.

Additionally or alternatively, the therapeutic polypeptide is an antibody or antibody-like molecule or comprises an antibody or antibody-like molecule specifically binding agonistically or antagonistically to at least one member of the group consisting of: PD-L1, TNFα, TGFβ, histone, IFNα, IFNβ, IFNγ, CXCL10, CX3C chemokine receptor 1 (CX3CR1), CTLA-4, programmed cell death protein 1 (PD-1), PD-1L, OX40, CD3, CD19, CD20, CD25, CD27, CD28, CD40, CD47, CD95, CD96, CD111, CD133, TREM2, IL-1, IL-1R, IL-4RA, IL-6, IL-10, IL-10R, IL-12, IL-12Rb1, IL-13Rα2, IL-23, Nogo-A, EGFR, EGFRvIII, Her2, PDGFR, TGFR, FGFR, TfR, LfR, IR, LDL-R, LRP-1, VEGFR, VEGF-A, Ang-2, α-synuclein, CSF1R, GITR, GITR, TIM-3, LAG-3, TIGIT, BTLA, VISTA, 4-1BB, B7-H3, CCL2, EphA2, EphA3, EphB2, EphB3, EphB4, LINGO-1, L1CAM, NCAM, SOD-1, SIGMAR-1, SIGMAR-2, TDP-43, Aβ, Tau, TRPM4, ASIC1, VGCCs, CB1, TTR, HTT, JCV, C9orf72, and albumin.

The possible therapeutic polypeptides mentioned are described in more detail in WO 2020/201167 A1 (UNIV ZUERICH, 08 October 2020) and WO 2020/201168 A1 (UNIV ZUERICH, 08 October 2020) from the prior art. Reference is therefore made to these documents. In addition, further therapeutic polypeptides according to the invention are possible, which are mentioned in these documents, for example. The interleukins may be human interleukins (e.g. hIL-12). The therapeutic polypeptide from the preceding list comprises or is linked to an Fc-region of IgG, an FcRn-binding region of albumin, and/or an albumin-binding region. For example, IL-12 can be fused to the Fc region of IgG to produce IL-12Fc, which would be the polypeptide according to the invention. The wild-type variants or modified variants can be considered. Regarding the antibody or antibody-like molecule specifically binding agonistically or antagonistically to at least one member of the above list, for example, an anti-CTLA-4 monoclonal antibody (mAb) and/or a CD40 agonistic biologic can be comprised in the therapeutic polypeptide.

Further therapeutic polypeptides with an Fc region of IgG include alemtuzumab (CAS-No.: 216503-57-0), which is used to treat multiple sclerosis.

Therapeutic polypeptides with an FcRn-binding region of albumin include
- WTX-330 (NIRSCHL, C. J. et al., mWTX-330, an IL-12 INDUKINE Molecule, Activates and Reshapes Tumor-Infiltrating CD8+ T and NK Cells to Generate Antitumor Immunity, Cancer Immunol. Res. 2023, Vol. 11, No. 7, pages 962 to 977),
- nab-PTX (albumin-bound paclitaxel; e.g. available under the brand name abraxane; paclitaxel: CAS-No. 33069-62-4), and
- CLN-617 (Clinical trial No. NCT06035744; MEHTA, N. K. et al. CLN-617 retains IL-2 and IL-12 in injected tumors to drive robust and systemic immune-mediated antitumor activity. Cancer Immunol. Res. 2024, https://doi.org/10.1158/2326-6066.CIR-23-0636).

Therapeutic polypeptides with an albumin-binding region include
- n501-aHSA-MMAE (LI, Q. et al. Half-life extension of single-domain antibody-drug conjugates by albumin binding moiety enhances antitumor efficacy. MedComm. 2024, Vol. 5, article 557),
- SON-1010 (KENNEY, R. T. et al. A phase I trial of SON-1010, a tumor-targeted, interleukin-12-linked, albumin-binding cytokine. Front. Immunol. 2024, Vol. 15, article 1362775),
- MP0317 (synonyms: anti-CD40/anti-FAP DARPin agent MP0317, FAP x CD40-targeting DARPin MP0317, FAP/CD40-targeting designed ankyrin repeat protein MP0317; RIGAMONTI, N. et al. A Multispecific Anti-CD-40 DARPin Construct Induces Tumor-Selective CD40 Activation and Tumor Regression, Cancer Immunol. Res. 2022, Vol. 10, No. 5, pages 626 to 640), and
- MP0533 (synonyms: CD33/CD123/CD70-targeting DARPin MP0533, DARPin T-cell engager MP0533, multispecific DARPin CD3 engager targeting CD33/CD123/CD70 MP0533; PABST, T. et al. MP0533, a CD3-Engaging Darpin Targeting CD33, CD123, and CD70 in Patients with Relapsed/Refractory AML or MDS/AML: Preliminary Results of a Phase 1/2a Study, blood 2023, Vol. 142, Suppl. 1, page 2921).

Human serum albumin (HSA)-binding Designed Ankyrin Repeat Proteins (DARPins) (such as n501-aHSA-MMAE, SON-1010, MP0317 and MP0533) as well as their function are further described in HOEF-MAN, S. et al. (Pre-Clinical Intravenous Serum Pharmacokinetics of Albumin Binding and Non-Half-Life Extended Nanobodies, Antibodies 2015, Vol. 4, pages 141 to 156) and in STEINER, D. et al. (Half-life extension using serum albumin-binding DARPin domains, PEDS 2017, Vol. 30, No. 9, pages 583 to 591).

In another preferred embodiment of any aspect of the invention, the FcRn inhibitor is delivered by at least one mode of systemic administration selected from the group consisting of:
- by intravenous administration,
- by intraperitoneal administration,
- by transdermal administration,
- by oral administration, and
- by intra-arterial administration, and
- by inhalative administration.

Preferably, it is delivered by intravenous administration.

In a further preferred embodiment of any aspect of the invention the therapeutic polypeptide is delivered by at least one mode of localised central nervous system administration selected from the group consisting of:
- by intracerebral administration,
- by intraparenchymal administration,
- by intrathecal administration,
- by intraocular administration,
- by intraventricular administration,
- by epidural administration,
- by convection-enhanced delivery,
- by in situ production of the therapeutic polypeptide,
- by release from implanted slow release formulations,
- by molecular transport into the CNS,
- by cellular transport into the CNS,
- by intranasal administration,
- by administration into brain tumour, and
- by administration into brain tumour resection cavity walls;
preferably by intraparenchymal administration and/or convection-enhanced delivery.

In a further preferred embodiment of any aspect of the present invention, the FcRn inhibitor is administered prior to the therapeutic polypeptide, preferably the FcRn inhibitor is administered at least 3 h, preferably at least 6 h, more preferably at least 12 h, even more preferably at least 24 h, even more preferably at least 3 days, even more preferably at least 1 week, even more preferably at least 2 weeks, most preferably at least 1 month prior to the administration of the therapeutic polypeptide.

It was found that the administration of the FcRn inhibitor prior to the administration of the therapeutic polypeptide leads to an effective FcRn blockade.

In an alternative preferred embodiment, the FcRn inhibitor is administered essentially at the same time as the therapeutic polypeptide. This means that the time difference between the administration of the FcRn inhibitor and the therapeutic polypeptide is less than 60 min, preferably at most 30 min, more preferably at most 15 min, most preferably at most 5 min. If the first component is administered over a period of time, the second component may be administered within this period. In yet another alternative preferred embodiment, the FcRn inhibitor is administered after the therapeutic polypeptide. In this case, preferably, the FcRn inhibitor is administered at least 1 h, preferably at least 3 h, more preferably at least 6 h, even more preferably at least 12 h, most preferably at least 24 h after the administration of the therapeutic polypeptide. The choice of the appropriate sequence and time intervals between administrations depends on the FcRn inhibitor used, the therapeutic polypeptide used and the routes of administration. The skilled person is familiar with how to assess and set these parameters.

Clinical dosages of the FcRn inhibitor and the therapeutic polypeptide depend on the specific compounds and disease to be treated. For example, dosage information on the known FcRn inhibitors efgartigimod alfa and rozanolixizumab are known from the prior art in ZHU, L. N. et al. (FcRn inhibitors: a novel option for the treatment of myasthenia gravis. Neural. Regen. Res. 2023, Vol. 18, No. 8, pages 1637 to 1644).

The invention will now be described by way of more specific embodiments. These embodiments are not intended to limit the gist of the invention in any way, but rather serve to ease understanding of the invention.

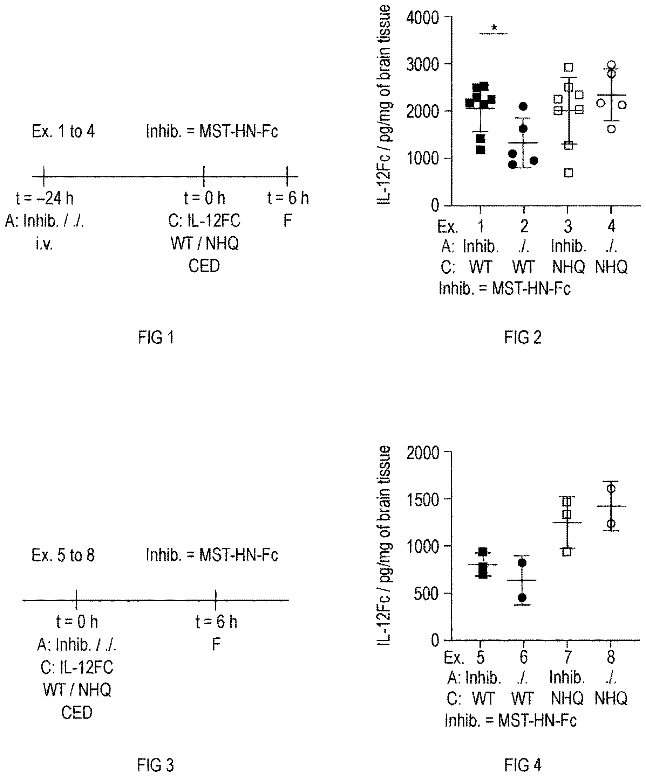

- Fig. 1:: Examples 1 to 4; timeline of the administration of the FcRn inhibitor (A; MST-HN-Fc (Inhib.) or control (./.)), the administration of the therapeutic polypeptide (C; IL-12Fc wild type (WT) or modified (NHQ)), and euthanasia and subsequent assessment of IL-12Fc levels in the brain tissue (F);
- Fig. 2:: Examples 1 to 4; IL-12Fc levels per mg of brain tissue in the ipsilateral hemisphere of hFcRn Tg32 mice 6 h after intraparenchymal administration of IL-12Fc WT and NHQ variants (C; therapeutic polypeptide) upon i.v. MST-HN-Fc administration (A; FcRn inhibitor, Inhib.) 24 h prior to administration of the therapeutic polypeptide; data shows mean ± SD;
- Fig. 3:: Examples 5 to 8; timeline of the administration of the FcRn inhibitor (A; MST-HN-Fc (Inhib.) or control (./.)), the administration of the therapeutic polypeptide (C; IL-12Fc wild type (WT) or modified (NHQ)), and euthanasia and subsequent assessment of IL-12Fc levels in the brain tissue (F); and
- Fig. 4:: Examples 5 to 8; IL-12Fc levels per mg of brain tissue in the ipsilateral hemisphere of hFcRn Tg32 mice 6 h after intraparenchymal co-administration of IL-12Fc WT and NHQ variants (C; therapeutic polypeptide) and MST-HN-Fc administration (A; FcRn inhibitor, Inhib.); data shows mean ± SD.

As an exemplary FcRn inhibitor, a biosimilar of efgartigimod alfa (MST-HN-Fc, a modified human IgG1 Fc fragment,) was used, together with IL-12Fc as an exemplary therapeutic polypeptide comprising an Fc region of IgG. According to multiple preclinical cancer models, IL-12 and the fusion polypeptide IL-12Fc are potentially suitable for treating cancer, including glioma. Embodiments according to the invention are shown in examples 1 and 3 in Table 1. Examples 2 and 4 to 8 show comparative examples.

**Table 1**

| Example | | | | | | | | |
|---|---|---|---|---|---|---|---|---|
| | 1 | 2 | 3 | 4 | 5 | 6 | 7 | 8 |
| A | Inhib. | ./. | Inhib. | ./. | Inhib. | ./. | Inhib. | ./. |
| B | i.v. | ./. | i.v. | ./. | CED | ./. | CED | ./. |
| C | IL-12Fc WT | IL-12Fc WT | IL-12Fc NHQ | IL-12Fc NHQ | IL-12Fc WT | IL-12Fc WT | IL-12Fc NHQ | IL-12Fc NHQ |
| D | CED | CED | CED | CED | CED | CED | CED | CED |
| E | 24 h | ./. | 24 h | ./. | 0 h | ./. | 0 h | ./. |
| F | 6 h | 6 h | 6 h | 6 h | 6 h | 6 h | 6 h | 6 h |

The letters in the first column of Table 1 mean:
- A:: FcRn inhibitor (Inhib.; MST-HN-Fc);
- B:: FcRn inhibitor, mode of administration (i.v. = intravenously; CED = local central nervous system convection-enhanced delivery);
- C:: therapeutic polypeptide (WT = wild type; NHQ = mutations at I253N, H435Q);
- D:: therapeutic polypeptide, mode of administration;
- E:: time interval between administration of the inhibitor and administration of the therapeutic polypeptide; and
- F:: time interval between administration of the therapeutic polypeptide and determining IL-12Fc levels in the brain tissue.

### Experimental Details

Human IL-12Fc variants were expressed in HEK239T after calcium phosphate transient transfection or using suspension HEK293 cell cultures. Protein was purified from the cleared culture supernatants by affinity chromatography using Protein G sepharose (Bio-Vision Inc.), concentration and buffer exchange using spin columns with a 30 kDa molecular weight cut off membranes (Sartorius). After affinity chromatography, proteins were further purified using ion exchange chromatography using diethylaminoethylcellulose columns (GE) and size exclusion chromatography columns (Superose 6 Increase 10/300GL, GE) into the histidine buffer (20 mM histidine 150 mM NaCl 0.05% Tween20 pH=6.0). Peak fractions were pooled and concentrated with ultrafiltration spin columns (Sartorius). Protein purity was validated by SDS-PAGE electrophoresis and Coomassie staining (VWR Life Science). For further information on IL-12Fc WT and IL-12Fc NHQ, please refer to documents WO 2020/201167 A1 (UNIV ZUERICH, 08 October 2020) and WO 2020/201168 A1 (UNIV ZUERICH, 08 October 2020), in particular to the sequence listings, SEQ ID No. 001 (Fc WT) and SEQ ID No. 004 (NHQ).

A biosimilar of efgartigimod alfa (MST-HN Fc with 'Abdeg' mutations M252Y/S254T/T256E/H433K/N434F (see ULRICHTS, P. et al. Neonatal Fc receptor antagonist efgartigimod safely and sustainable reduces IgGs in humans. J. Clin. Invest. 2018, Vol. 128, No. 10, pages 4372 to 4386; and VACCARO, C. J. et al. Engineering the Fc region of immunoglobulin G to modulate in vivo antibody levels. Nat. Biotechnol. 2005, Vol. 23, pages 1283 to 1288) and starting at residue D221 in the hinge region, EU numbering), were produced using pcDNA3.4 expression plasmid (Thermo Fisher Scientific) transfected (Expifectamine, Transfection Kit; Thermo Fisher Scientific) into suspension HEK293 cultures (Expi293F, Thermo Fisher Scientific), followed by protein A sepharose affinity chromatograph. Aggregates were removed using size exclusion chromatography. The DrugBank Accession Number of efgartigimod alfa is DB15270.

B6.Cg-Fcgrttm1Dcr Tg (FCGRT)32Dcr/DcrJ (hFcRn Tg32) mice were from The Jackson Laboratory (stock number 014565) and bred in-house. All efforts were made to minimize the number of animals used and their suffering. Euthanasia was performed by controlled CO₂ asphyxiation.

CED into the brain was performed as previously published (BEFF-INGER, M. et al. Delivery of Antibodies into the Murine Brain via Convection-enhanced Delivery. J. Vis. Exp. 2019, Vol. 149, e59675). Briefly, mice were injected using a Hamilton syringe 10 µl (Hamilton) with catheters made using a 27 G blunt-end needle (Hamilton) with a 1 mm step at the tip made of fused silica with internal diameter of 0.1 mm and wall thickness of 0.0325 mm (Postnova). For non-tumour-bearing mice a burr hole drilled at position 1 mm anteroposterior and 2 mm lateral of bregma. The catheter was lowered into the burr hole to a depth of 3.5 mm below the dura surface. Injection was performed in a volume of 1 µL at 0.2 µL/min, then 2 µL at 0.5 µL/min and 2 µL at 0.8 µL/min. After leaving the needle in place for 2 min, it was retracted at 1 mm/min. Injection mixes were prepared in PBS or histidine buffer containing 20 mM L-histidine (Sigma-Aldrich), 150 mM NaCl (Sigma-Aldrich), 0.05% Tween20 (Biosolve) pH=6.0.

For systemic administration of the FcRn inhibitor (examples 1 to 4), efgartigimod alfa was intravenously (i.v.) injected into hFcRn (human FcRn) Tg32 mice at of 100 µg per mouse. 24 hours post injection, CED was performed either with human IL-12Fc WT or IL-12Fc NHQ into the right striatum. 6 hours post-CED, the mice were euthanised, and plasma and brain tissue were collected for further analysis.

To study local influence of the FcRn inhibitor efgartigimod alfa (examples 5 to 8), hFcRn Tg32 animals were injected with 1 µg hIL-12Fc (human IL-12Fc) WT or NHQ into the right striatum of mice via CED in the absence or presence of 1 µg of efgartigimod. 6 hours post-CED, the mice were euthanised, and plasma and brain tissue were collected for further analysis.

For all examples, after euthanasia and removal of the skullcap, cerebellum and olfactory bulbs were discarded, the brain hemispheres were separated along the midline and the injected (ipsilateral) hemisphere was frozen at -20 °C. Brain lysates were prepared by homogenisation in ice cold lysis buffer (Cell Signaling) to a final concentration of 2x lysis buffer with protease inhibitors (Complete Mini Tablets, Roche). 0.1 mL of lysis buffer was added per 10 mg of brain tissue. Brain tissue was minced using scissors, then passed through a 20 G needle and finally sonicated for 20 seconds. Samples were spun down and supernatants were stored at -80 °C. Protein concentration was measured using Pierce BCA assay kit (Thermo Fisher Scientific) and this data was used to correct for the protein extraction efficiency within each experiment (corrected ELISA concentration = (ELISA concentration) * (average total protein concentration)/(total protein concentration of the sample)).

Samples were diluted in PBS containing 0.05% Tween-20 and 0.1% bovine serum albumin (BSA; Sigma-Aldrich) and IL-12 levels were assessed by ELISA for hIL-12p70 (Mabtech), blocking buffer PBS 0.05% Tween20 0.1% BSA (Sigma-Aldrich). The concentration in the brain was calculated by dividing the total amount of IL-12 extracted from the brain corrected for the efficacy of protein extraction by the weight of the hemisphere (pg/mg of brain tissue) .

For determining serum to brain or plasma to brain ratio (not done for the present examples, but mentioned above): for plasma isolation, blood is collected from vena cava after euthanasia in dried K₂EDTA tubes (BD), spun down 5 mins, 10'000 g and plasma is transferred and frozen in 96 well microplates (Abgene) at -20 °C. For serum isolation, blood is collected in a microcentrifuge tube and spun 5 mins at RT 10'000 g. Serum (supernatant) was transferred to a fresh 96-well microplate (Abgene) and frozen at -20 °C. The concentration of IL-12Fc in serum or plasma is described in pg/ml. To calculate the ratio, plasma concentration is described in pg/ml. whereas concentration in brain is calculated by dividing the total amount of protein of interest extracted from the brain corrected for the efficiency of protein extraction by the weight of the hemisphere (pg/mg of brain tissue) .

Equilibrium dissociation constants K_{D} can be determined using surface plasmon resonance (SPR) (not done for the present examples, but mentioned above). SPR can be performed using the ProteOn XPR36 System (Bio-Rad Laboratories, Inc.), coating human recombinant biotinylated FcRn (Immunitrack) on a ProteOn NLC sensor chip (Bio-Rad.). Coating density is 100-115 RU signal for antibodies and 250-260 RU for IL-12Fc constructs, for example. Analytes are injected in the range 0 nM to 6075 nM. Dissociation constants (K_{D}) are calculated based on the binding equilibrium analysis. Running buffer is histidine buffer (20 mM l-histidine, 150 mM NaCl, 0.05% Tween20, pH=6.0) and recovery buffer is 20mM Tris-HCl pH=7.5 with 150 mM NaCl 0.05% Tween20 (Merck). Thus, K_{D} values for the binding affinity to FcRn are indicated with FcRn in the immobilised phase.

### Results

In examples 1 to 4, efgartigimod alfa as the FcRn inhibitor (examples 1 and 3) or phosphate-buffered saline (PBS; examples 2 and 4) was administered intravenously 24 h before administering IL-12Fc as a therapeutic polypeptide by intraparenchymal CED to the central nervous system (Figure 1). In examples 1 and 2, wild type IL-12Fc was used that has a higher systemic efflux rate than IL-12 Fc NHQ, which bears modifications in its polypeptide sequence that results in a reduced affinity towards FcRn and thus in a reduced FcRn-mediated transcytosis through the blood-brain barrier into the blood.

Figure 2 shows the IL-12Fc levels in the brain tissue in the ipsilateral hemisphere of the test animals 6 h after administration of the therapeutic polypeptide (examples 1 to 4). Administering IL-12Fc WT without the FcRn inhibitor (example 2) results in a comparatively low IL-12Fc level in the brain. Systemic administration of the FcRn inhibitor prior to administration of the therapeutic polypeptide (example 1) results in significantly higher IL-12Fc levels in the brain. This indicates that FcRn blockade by intravenous administration of the inhibitor is effective and leads to lower systemic efflux of the therapeutic polypeptide. Example 4 further demonstrates that reducing the FcRn-affinity of the therapeutic polypeptide (IL-12Fc NHQ) leads to a similar retention of IL-12Fc in the brain as the use of an FcRn blocker administered systemically. This shows that complex modifications to the therapeutic polypeptide do not even have to be made and an FcRn inhibitor can be used instead. Administering the FcRn inhibitor intravenously followed by CED-administration of FcRn-silenced IL-12Fc NHQ (example 3) does not lead to an improved effect compared to examples 1 and 4.

Examples 5 to 8 show that local CED co-administration of the FcRn inhibitor and the therapeutic polypeptide, as illustrated in Figure 3, does not lead to the same result as systemic administration of the FcRn inhibitor. The experiments with the FcRn-silenced therapeutic polypeptide (IL-12Fc NHQ; examples 7 and 8) result in increased brain levels after 6 h compared to administration of the FcRn inhibitor with unmodified IL-12Fc (example 5). Comparing the levels between examples 5 and 6 and the levels between examples 1 and 2, the FcRn inhibitor has a lower retention effect when administered locally to the brain than when administered systemically. As discussed above, this indicates the importance of FcRn in the blood-brain barrier endothelium rather than in the parenchyma in mediating brain-to-blood export.

## Claims

1. An FcRn inhibitor, wherein FcRn is a neonatal fragment crystallisable receptor, for use in reducing, preferably preventing, the transfer, in particular the FcRn-mediated transcytosis, of a therapeutic polypeptide from the central nervous system into the systemic circulation,
- wherein the therapeutic polypeptide comprises at least one region selected from the group consisting of: an Fc-region of IgG, an FcRn-binding region of albumin, and an albumin-binding region, wherein the Fc-region is a crystallisable fragment region; and
- wherein the FcRn inhibitor is delivered by systemic administration and the therapeutic polypeptide is delivered by localised administration, preferably by localised central nervous system administration.

2. A therapeutic polypeptide comprising at least one region selected from the group consisting of: an Fc-region of IgG, an FcRn-binding region of albumin, and an albumin-binding region, wherein the Fc-region is a crystallisable fragment region, for use in a combination therapy in the prevention or treatment of a disease affecting the central nervous system, particularly a primary and secondary brain cancer,
- wherein the combination therapy further comprises an FcRn inhibitor, wherein FcRn is a neonatal fragment crystallisable receptor; and
- wherein the FcRn inhibitor is delivered by systemic administration and the therapeutic polypeptide is delivered by localised administration, preferably by localised central nervous system administration.

3. A combination of pharmaceutical components comprising:
- an FcRn inhibitor, wherein FcRn is a neonatal fragment crystallisable receptor; and
- a therapeutic polypeptide comprising at least one region selected from the group consisting of: an Fc-region of IgG, an FcRn-binding region of albumin, and an albumin-binding region, wherein the Fc-region is a crystallisable fragment region;
for use in a combination therapy in the prevention or treatment of a disease affecting the central nervous system, particularly a primary and secondary brain cancer, wherein FcRn inhibitor is delivered by systemic administration and the therapeutic polypeptide is delivered by localised administration, preferably by localised central nervous system administration.

4. The FcRn inhibitor for use according to claim 1, or the therapeutic polypeptide for use according to claim 2, or the combination for use according to claim 3, wherein the FcRn inhibitor is selected from the group consisting of:
- an FcRn-blocking antibody or antibody fragment;
- an FcRn-blocking peptide; and
- an FcRn-degrading agent, preferably an FcRn-degrading peptide, more preferably an FcRn-degrading proteolysis targeting chimera.

5. The FcRn inhibitor for use according to any one of claims 1 or 4, or the therapeutic polypeptide for use according to any one of claims 2 or 4, or the combination for use according to any one of claims 3 or 4, wherein the equilibrium dissociation constant K_{D} for the binding affinity for FcRn of the FcRn inhibitor
- at pH 6.0, is at most 30 nM, preferably is at most 25 nM, more preferably is at most 20 nM, most preferably is at most 15 nM; and/or
- at pH 7.4, is at most 360 nM, preferably is at most 350 nM, more preferably is at most 340 nM, most preferably is at most 330 nM.

6. The FcRn inhibitor for use according to any one of claims 1 or 4 to 5, or the therapeutic polypeptide for use according to any one of claims 2 or 4 to 5, or the combination for use according to any one of claims 3 to 5, wherein the equilibrium dissociation constant K_{D} for the binding affinity for FcRn of the therapeutic polypeptide
- at pH 6.0, is at most 750 nM, preferably is at most 650 nM, more preferably is at most 550 nM; and/or
- at pH 7.4, is at least 1000 nM, preferably is at least 1100 nM, more preferably is at least 1200 nM.

7. The FcRn inhibitor for use according to any one of claims 1 or 4 to 6, or the therapeutic polypeptide for use according to any one of claims 2 or 4 to 6, or the combination for use according to any one of claims 3 to 6, wherein the equilibrium dissociation constant K_{D} for the binding affinity for FcRn of the therapeutic polypeptide, at pH 6.0, is at least 5 times higher, preferably at least 10 times higher, most preferably at least 15 times higher than the equilibrium dissociation constant K_{D} for the binding affinity for FcRn of the FcRn inhibitor.

8. The FcRn inhibitor for use according to any one of claims 1 or 4 to 7, or the therapeutic polypeptide for use according to any one of claims 2 or 4 to 7, or the combination for use according to any one of claims 3 to 7, wherein the FcRn is endothelial FcRn at a barrier selected from the group consisting of:
- a blood-brain barrier,
- a barrier between spinal cord parenchyma and surrounding vasculature,
- a cerebrospinal fluid-brain barrier,
- a blood-cerebrospinal fluid barrier, and
- a blood-tumour barrier;
preferably a blood-brain barrier.

9. The FcRn inhibitor for use according to any one of claims 1 or 4 to 8, or the therapeutic polypeptide for use according to any one of claims 2 or 4 to 8, or the combination for use according to any one of claims 3 to 8, wherein the FcRn inhibitor reduces, preferably substantially prevents, the binding of the therapeutic polypeptide with FcRn, preferably at a pH of at most 6.0.

10. The FcRn inhibitor for use according to any one of claims 1 or 4 to 9, or the therapeutic polypeptide for use according to any one of claims 2 or 4 to 9, or the combination for use according to any one of claims 3 to 9, wherein the FcRn inhibitor is selected from the group consisting of:
- an antibody or antibody fragment comprising an Fc-region of IgG, wherein the Fc-region is a crystallisable fragment region, comprising a modification resulting in enhanced affinity to the FcRn, preferably, the antibody or antibody fragment is a human IgG1 Fc fragment with MST-HN mutations, more preferably the antibody or antibody fragment is efgartigimod alfa or a biosimilar thereof;
- rozanolixizumab;
- batoclimab;
- nipocalimab;
- SYN1436;
- SYN1327; and
- SYNT001.

11. The FcRn inhibitor for use according to any one of claims 1 or 4 to 10, or the therapeutic polypeptide for use according to any one of claims 2 or 4 to 10, or the combination for use according to any one of claims 3 to 10, wherein the therapeutic polypeptide is selected from the group consisting of or comprises a member of the group consisting of:
- an immunomodulatory agent, preferably a cytokine, more preferably an interleukin or interleukin derivative, preferably selected from the group consisting of IL-12Fc and IL-10Fc;
- an interferon;
- an agonist of a tumour necrosis factor receptor superfamily member;
- a transforming growth factor;
- an immune checkpoint and regulatory protein;
- a colony-stimulating factor;
- a costimulatory molecule;
- a chemokine;
- a growth factor; and
- a neurotrophic factor.

12. The FcRn inhibitor for use according to any one of claims 1 or 4 to 11, or the therapeutic polypeptide for use according to any one of claims 2 or 4 to 11, or the combination for use according to any one of claims 3 to 11, wherein
- the therapeutic polypeptide is selected from the group consisting of or comprises a member of the group consisting of: IL-1RA, IL-2, IL-4, IL-5, IL-6, IL-7, IL-8, IL-10, IL-12, IL-13, IL-15, IL-18, IL-21, IL-23, IL-35, interferon alpha (IFNα), interferon beta (IFNβ), interferon gamma (IFNγ), tumour necrosis factor alpha (TNFα), transforming growth factor alpha (TGFα), transforming growth factor beta (TGFβ), transforming growth factor beta receptor II (TGFβRII), signal regulatory protein alpha (SIRPα), granulocyte-macrophage colony-stimulating factor (GM-CSF), granulocyte-colony stimulating factor (G-CSF), CD80, CD86, CD95, GITRL, 4-1BBL, OX40L, EphrinA1, EphrinB2, EphrinB5, C-X-C motif chemokine 10 (CXCL10), CX3CL1, CXCL16, BDNF, GDNF, NRTN, ARTN, PSPN, CNTF, TRAIL, CCL5, CCL10, CCL16, CCL21, IgG1, IgG2, IgG3, IgG4, and albumin, preferably IL-12; and/or
- the therapeutic polypeptide is an antibody or antibody-like molecule or comprises an antibody or antibody-like molecule specifically binding agonistically or antagonistically to at least one member of the group consisting of: PD-L1, TNFα, TGFβ, histone, IFNα, IFNβ, IFNγ, CXCL10, CX3C chemokine receptor 1 (CX3CR1), CTLA-4, programmed cell death protein 1 (PD-1), PD-1L, OX40, CD3, CD19, CD20, CD25, CD27, CD28, CD40, CD47, CD95, CD96, CD111, CD133, TREM2, IL-1, IL-1R, IL-4RA, IL-6, IL-10, IL-10R, IL-12, IL-12Rb1, IL-13Rα2, IL-23, Nogo-A, EGFR, EGFRvIII, Her2, PDGFR, TGFR, FGFR, TfR, LfR, IR, LDL-R, LRP-1, VEGFR, VEGF-A, Ang-2, α-synuclein, CSF1R, GITR, GITR, TIM-3, LAG-3, TIGIT, BTLA, VISTA, 4-1BB, B7-H3, CCL2, EphA2, EphA3, EphB2, EphB3, EphB4, LINGO-1, L1CAM, NCAM, SOD-1, SIGMAR-1, SIGMAR-2, TDP-43, Aβ, Tau, TRPM4, ASIC1, VGCCs, CB₁, TTR, HTT, JCV, C9orf72, and albumin.

13. The FcRn inhibitor for use according to any one of claims 1 or 4 to 12, or the therapeutic polypeptide for use according to any one of claims 2 or 4 to 12, or the combination for use according to any one of claims 3 to 12, wherein the FcRn inhibitor is delivered by at least one mode of systemic administration selected from the group consisting of:
- by intravenous administration,
- by intraperitoneal administration,
- by transdermal administration,
- by oral administration,
- by intra-arterial administration, and
- by inhalative administration;
preferably by intravenous administration.

14. The FcRn inhibitor for use according to any one of claims 1 or 4 to 13, or the therapeutic polypeptide for use according to any one of claims 2 or 4 to 13, or the combination for use according to any one of claims 3 to 13, wherein the therapeutic polypeptide is delivered by at least one mode of localised central nervous system administration selected from the group consisting of:
- by intracerebral administration,
- by intraparenchymal administration,
- by intrathecal administration,
- by intraocular administration,
- by intraventricular administration,
- by epidural administration,
- by convection-enhanced delivery,
- by in situ production of the therapeutic polypeptide,
- by release from implanted slow release formulations,
- by molecular transport into the CNS,
- by cellular transport into the CNS,
- by intranasal administration,
- by administration into brain tumour, and
- by administration into brain tumour resection cavity walls;
preferably by intraparenchymal administration and/or convection-enhanced delivery.

15. The FcRn inhibitor for use according to any one of claims 1 or 4 to 14, or the therapeutic polypeptide for use according to any one of claims 2 or 4 to 14, or the combination for use according to any one of claims 3 to 14, wherein the FcRn inhibitor is administered prior to the therapeutic polypeptide, preferably the FcRn inhibitor is administered at least 3 h, preferably at least 6 h, more preferably at least 12 h, even more preferably at least 24 h, even more preferably at least 3 days, even more preferably at least 1 week, even more preferably at least 2 weeks, most preferably at least 1 month prior to the administration of the therapeutic polypeptide.
